Europäisches Patentamt

European Patent Office

Office européen des brevets

(11) Publication number: **0 169 225**

**B1**

## (12) EUROPEAN PATENT SPECIFICATION

(45) Date of publication of patent specification: **01.06.88**

(21) Application number: **85900699.1**

(22) Date of filing: **17.01.85**

(86) International application number:
**PCT/GB85/00022**

(87) International publication number:
**WO 85/03308 01.08.85 Gazette 85/17**

(51) Int. Cl.⁴: **C 12 Q 1/00**

(54) MONITORING MEMBRANE BOUNDED SYSTEMS.

(30) Priority: **21.01.84 GB 8401638**

(43) Date of publication of application:
**29.01.86 Bulletin 86/05**

(45) Publication of the grant of the patent:
**01.06.88 Bulletin 88/22**

(84) Designated Contracting States:
**AT BE CH DE FR GB LI NL SE**

(56) References cited:
**EP-A-0 028 793**
**WO-A-82/04264**
**AU-B-2 060 876**

**Chemical Abstracts, vol. 95, no. 17, 26 October 1981 (Columbus, Ohio, US). P.R. RICH: "A generalised model for the equilibration of quinone peels with their biological donors and acceptors in membrane-bound electron transfer chains", see page 185, colun 1, abstract no. 145423m, & FEBS Lett. 1981, 173-178 (Eng.)**

(73) Proprietor: **The British Petroleum Company p.l.c.**
**Britannic House Moor Lane**
**London EC2Y 9BU (GB)**

(72) Inventor: **RICH, Peter, Ronald**
**75 Kingston Street**
**Cambridge CB2 1NU (GB)**

(74) Representative: **Ryan, Edward Terrence et al**
**BP INTERNATIONAL LIMITED, Patents Division**
**Chertsey Road**
**Sunbury-on-Thames Middlesex, TW16 7LN (GB)**

(56) References cited:
**Chemical Abstracts, vol. 96, no. 15, 12 April 1982 (Colmbus, Ohio, US). F.L. CRANE et al.: "Requirements for coenzyme Q10 for transmembrane electron transport: microbial, mitochondrial and Golgi membranes", see page 281, column 1, abstract no. 118067n, & Biomed. Clin. Aspects Coenzyme Q, 1981, 183-190 (Eng.)**

Courier Press, Leamington Spa, England.

# Description

The present invention relates to the monitoring of electrochemical reactions taking place within membrane-bounded biochemical systems such as the living cell. In the case of eukaryotic cells, other membrane-bounded organelles are contained within the outer cellular membrane e.g. the mitochondria responsible for respiration and chloroplasts responsible for photosynthesis in photosynthetic organisms. These subsystems can be isolated from the cell and studied separately from it. However, it is often desirable to study them *in situ* in an intact functioning cell. One way of monitoring the function of a membrane-bounded process is by observing the concentration in a medium surrounding the membrane of reactants required or products released into this medium by the system of interest. To study these from a system which is still present within the intact functioning cell is often not possible because the cellular membrane presents a barrier between the metabolite and the detecting instrument.

Living cells are used in a variety of industrial processes e.g. the production of ethanol by yeast, the production of antibiotics by micro-organisms or the testing of herbicides on plant cells. Any method which enables a manufacturer to follow more directly what is happening within a membrane-bounded system is of great value in making possible a closer control of the manufacturing process or in the testing of the effects of new chemicals on various biological processes, or in assessing the vigour or health of the biological organism of which the system forms part. We have now found a way of monitoring certain biochemical reactions taking place within membrane-bounded systems either in isolated organelles or in situ within the cell.

Chemical Abstract 95:145423M proposes a model for the equilibration of quinone pools in biochemical systems but does not suggest the use of a reactant mimic which can pass through the membrane of a biological system.

Chemical Abstract 96:118067M discusses transmembrane electron transport involving ferricyanide ion. This ion is not capable of passing through the membrane.

WO—A 82/04264, AU—B—20608/76, and EP 28 793 also contain no disclosure relevant to the use of materials able to pass across membranes in order to study reactions occurring inside a membrane-bounded system.

According to the present invention, the process for monitoring a reaction taking place in a membrane bounded biochemical system comprises introducing a membrane-passing reactant mimic which is a molecule capable of undergoing an electrochemical reaction into a medium in contact with the system and observing changes in the reactant mimic in the medium with an electrode.

By "reactant mimic" we mean a compound which is capable of undergoing the specific reaction of interest in a way similar to the natural reactant. Unlike the natural reactant, however, the reactant mimic is able to pass across membranes and react both with the biochemical site and with the detecting electrode. By this means it is possible to gain information on a specific reaction occurring inside the cell with an external monitoring device which is able to monitor changes in the reactant mimic.

The reactant mimic used depends on the reaction to be studied. Selection of a suitable chemical requires an understanding of the biochemical reaction mechanism in order to obtain the degree of specificity required. In principle, a very wide range of bioelectrochemical reactions can be monitored in this way, although in each case a knowledge of the biochemistry of the targeted site and of the electrochemistry of the reactant mimic will be required.

For example, the present invention may be applied to the study of specific reactions within mitochondria and chloroplasts. These organelles may be studied separated from the cell so as to give partially simplified systems. However, it has also proved possible to monitor the same reactions when the organelles are still in situ in the intact functioning cell. The intact cell may be studied as a suspension in an aqueous medium. Thus suspensions of bacteria, algae, and yeasts have been studied in this way. Connected cell tissue (e.g. both normal plant tissue and callus tissue) as well as suspensions of cells, may be studied in this way by putting an electrode in an aqueous medium in contact with the cell walls of the tissue. In general, biochemical reactions tends to be more complicated when still inside the cell presumably because of additional control mechanisms which are operative.

The external medium in contact with the membrane bounded system to be studied is selected so as not to destroy the membrane-bounded system to be studied is selected so as not to destroy the membrane-bounded system e.g. by osmosis or by containing toxic materials which can pass across the medium or will adversely affect the reactant mimic or the means used to observe the reactant mimic. Liquid media suitable for dispersing membrane systems without adversely affecting their function are known. Such media will normally be aqueous.

Requirements for selection as a reactant mimic are:

(a) it must react rapidly only with the site of interest;

(b) it must react reversibly at the site of interest;

(c) it must have a partition coefficient between water and lipid within certain limits so that it can pass across membranes and yet still spend significant times in the aqueous media;

(d) it must be able to react reversibly with the electrode used to observe it in the medium;

(e) it must be usable in extremely low concentrations so that the functioning of the biological system is unaffected by its presence.

Examples of compounds which may be used as reactant mimics are the quinones (or the corresponding reduced form namely the quinols). A quinone system which reacts specifically with an intracellular metabolic process can be used. In reacting with the biological system, the ratio of the oxidised to reduced forms changes in an amount dependent upon the activity of the particular metabolic process in question, and the change can be monitored electrochemically as described above. By altering the physical properties of the quinone so that it can pass across cellular membranes, the technique becomes applicable to intact cellular systems as well as to isolated cellular subfractions. Specific examples of substituted p-benzoquinones which can be used are given below.

Two specific reactions which may be studied with the technique of the present invention take place in mitochondria and chloroplasts respectively. We have found that chemicals such as herbicides can have a profound effect on these reactions. In mitochondria, changes of the rate of flow into and the rate of flow out of the endogenous ubiquinone-10 component of the respiratory chain may be studied. For this purpose we have used the reactant mimics ubiquinone-1 or tetramethyl-p-benzoquinone. Ubiquinone-1 is 2,3-dimethoxy-5-methyl-6-isoprenyl-p-benzo-quinone.

In chloroplasts, changes in the endogenous plastoquinone-9 pool which is operative between the two different types of light energy-trapping photosystems may be studied. For this purpose we have used the reactant mimics plastoquinone-1, plastoquinone-2 and trimethyl-p-benzoquinone. Plastoquinone-1 is 2,3-dimethyl-5-isoprenyl-p-benzoquinone and plastoquinone-2 is 2,3-dimethyl-5-geranyl-p-benzoquinone.

These are chosen on the basis of the above listed criteria with the additional feature that their thermodynamic midpoint potentials are such that they are expected to change their redox states significantly as the endogenous components change theirs.

A suitable method of observing changes in the reactant mimic in the medium in contact with the membrane-bounded system is to measure the redox state of the mimic using for example a glassy carbon working electrode set at appropriate potential.

Thus *para*-Benzoquinones can interact with electrode surfaces in such a way that electrons are passed between the quinone or quinol and the electrode. For mechanistic reasons, however, the reaction is "electrochemically irreversible" with the oxidative and reductive waves being quite separated and bearing little relation to the overal equilibrium thermodynamic potential of the quinone system. This means that a potentiometric technique cannot be used to monitor the ratio of oxidised and reduced quinone present in a particular medium. Instead, an amperometric technique is used. The electrode can be set at a potential which monitors only oxidised quinone or only reduced quinone. For example, at pH 7 by setting the potential of glassy carbon to −250 mV versus SHE (Standard Hydrogen Electrode) a current flows which is proportional to ubiquinone-1 concentration. Alternatively, a potential of +250 mV can be used to monitor ubiquinol-1 only and ubiquinone-1 is then without effect.

A current which is proportional to either the quinol or quinone concentration will flow between a carbon electrode and a platinum wire counter electrode. A reference voltage is provided by a silver/silver chloride reference electrode. Changes in redox state of the mimic are then monitored by changes in current flow between working and counter electrodes. The solution is stirred and currents of the order of 1—10 nanoamps are generally encountered. The concentration of mimic used is in the range of 1—500 nanomolar, although this can be decreased further with more sensitive amplification of the signal.

In the examples given below, a three electrode configuration (working, counter and reference electrode) has been used. For many applications however, where small changes in working electrode potential do not significantly affect current flow (i.e. where there is only a small net current), a two electrode configuration (working and reference electrodes only) is usable. Other electrochemical techniques may be suitable in certain situations. When diffusion into/out of cells is very slow, then single sweep voltammetry can be used to give a measure of steady state redox poise after suitable time has elapsed for equilibrium to occur. In other cases, a rotating ring disc electrode could be used to generate quinone or quinol and to determine how much of the generated species is used up by the biological system before the generated species reaches the ring electrode.

The invention will now be illustrated by reference to the following Examples.

### Example 1

Isolated Rat Liver mitochondria were prepared by conventional methods and resuspended in a liquid medium containing 100 mM KCl, 1 mM EDTA (ethylene diamine tetraacetate) and 10 mM Potassium Phosphate buffer pH 7.2. The concentration of Rat Liver mitochondria was 2 mg protein/ml.

A sample of the above mixture was placed in a glass vessel, containing two electrodes, a glassy carbon electrode and a platinum wire electrode. A silver/silver chloride half cell in liquid contact with the contents of the vessel provides a reference electrode. The carbon electrode is the working electrode and is set at a potential higher than the peak current potential for the conversion of ubiquinol-1 to ubiquinone-1. The potential applied to the carbon electrode was thus + 370 mV in relation to the silver/silver chloride reference.

290 nM of ubiquinol-1 was added and the current flowing between the glassy carbon electrode and the platinum electrode was recorded while the liquid medium was stirred.

10 mM of pyruvate, a mitochondrial fuel was added, followed by 400 µM of adenosine diphosphate (ADP).

Sharp changes in the current were produced each time the above materials were introduced. After the addition of ADP the current remained substantially constant for several minutes before starting to rise. The period of constant current is interpreted as corresponding to the conversion of ADP to ATP (adenosine triphosphate).

The results obtained show that reactions inside the mitochondria can be followed by external electrodes in accordance with the present invention.

In the absence of the mitochondria no changes in current would have been observed on addition of the pyruvate or adenosine diphosphate.

Example 2

An experiment was carried out as in Example 1 but using a suspension of pea chloroplasts in a medium with a pH 7.4 containing 1 mM methyl viologen as a terminal electronic acceptor. The concentration of pea chloroplasts was 0.22 mg/ml.

The reaction mimic used was plastoquinol-1.

Addition of plastoquinol-1 causes a sharp increase in current followed by a fall. Illumination of the vessel with light with a wavelength of 645 nm caused a sharp increase in current to a steady high level. Addition of adenosine diphosphate (ADP) (400 µM) caused an increase in current followed by a decrease, which is attributed to the conversion of all the ADP to adenosine triphosphate (ATP). Addition of ammonium chloride ($NH_4Cl$) which is known to interfere with the charged state of the chloroplast caused a marked change in the current.

The results obtained clearly show that reactions taking place only within the chloroplasts affect the current between the electrodes in the medium outside the electrodes.

Example 3

An experiment was carried out as in Example 1 but using a suspension of intact cells of the alga, *Phormjdium laminosum*, in a buffer containing plastoquinone-1. Subjecting the vessel to light at 715 nm caused a decrease in current between the electrodes. The current increased again when the light was switched off.

Example 4

An experiment was carried out as in Example 1 but using intact cells of the bacterium *Rhodopseudomonas sphaeroides* in a buffer of pH 7.2 containing ubiquinone-1. Subjecting the vessel to light at 610 nm caused an initial small rise in current followed by a substantial fall. When the light was switched off the current continued to decrease for a short time and then began to increase again.

The photochemical reactions taking place inside the intact bacterium are being detected by their effect on the redox reactions of ubiquinol-1/ ubiquinone-1 at the electrode in the external liquid medium.

In addition to the above examples similar experiments have been carried out using protoplast cells from barley and yeast cells.

In addition an experiment has been carried out using intact pea leaves i.e. connected cell tissue. The cuticle was removed and the electrodes placed on the exposed surface.

**Claims**

1. The process for monitoring a reaction taking place in a membrane bounded biochemical system which comprises introducing a membrane-passing reactant molecule capable of undergoing an electrochemical reaction into a medium in contact with the system and observing changes in the reactant mimic in the medium with an electrode.

2. The process according to claim 1 wherein the electrochemical reaction is a redox reaction.

3. The process according to claim 2 wherein the reactant mimic is a quinone or quinol.

4. The process according to any one of the preceding claims wherein the quinone is ubiquinone-1, plastoquinone-1, or the corresponding quinols.

5. The process according to any one of the preceding claims wherein the changes in the reactant mimic are observed by measuring the current passing through the electrode.

6. The process according to any one of the preceding claims wherein the electrode is immersed in a suspension of mitochondria or chloroplasts.

7. The process according to any one of the preceding claims wherein the electrode is immersed in a suspension of intact cells.

8. The process according to any one of the preceding claims wherein the electrode is placed in a medium in contact with connected cell tissue.

**Patentansprüche**

1. Verfahren zur Überwachung einer in einem membrangebundenen biochemischen System stattfindenden Reaktion durch Einführen eines durch die Membran hindurchtretenden Moleküls eines Reaktanden, das befähigt ist, eine elektrochemische Reaktion einzugehen, in ein in Kontakt mit dem System befindliches Medium und Beobachten der Veränderungen in dem Reaktanden-Simulanten in dem Medium mittels einer Elektrode.

2. Verfahren nach Anspruch 1, worin die elektrochemische Reaktion eine Redox-Reaktion ist.

3. Verfahren nach Anspruch 2, worin der Reaktanden-Simulant ein Chinon oder Hydrochinon ist.

4. Verfahren nach irgendeinem der vorhergehenden Ansprüche, worin das Chinon Ubichinon-1, Plastochinon-1 oder eines der entsprechenden Hydrochinone ist.

5. Verfahren nach irgendeinem der vorhergehenden Ansprüche, worin die Veränderungen in dem Reaktanden-Simulanten durch Messen des durch die Elektrode hindurchgehenden Stromes beobachtet werden.

6. Verfahren nach irgendeinem der vorhergehenden Ansprüche, worin die Elektrode in eine Suspension von Mitochondrien oder Chloroplasten getaucht wird.

7. Verfahren nach irgendeinem der vorhergehenden Ansprüche, worin die Elektrode in ein Suspension intakter Zellen getaucht wird.

8. Verfahren nach irgendeinem der vorhergehenden Ansprüche, worin die Elektrode in einem Medium in Kontakt mit Bindegewebszellen angeordnet wird.

**Revendications**

1. Procédé de contrôle d'une réaction qui se déroule dans un système biochimique délimité par une membrane, qui comprend l'introduction d'une molécule d'un réactif traversant une membrane, capable de présenter une réaction électrochimique dans un milieu placé au contact du système, et l'observation des changements du substitut de réactif dans le milieu, à l'aide d'une électrode.

2. Procédé selon la revendication 1, dans lequel la réaction électrochimique est une réaction redox.

3. Procédé selon la revendication 2, dans lequel le substitut de réactif est une quinone ou un quinol.

4. Procédé selon l'une quelconque des revendications précédentes, dans lequel la quinone est l'ubiquinone-1, la plastoquinone-1 ou les quinols correspondants.

5. Procédé selon l'une quelconque des revendications précédentes, dans lequel les modifications du substitut de réactif sont observées par mesure du courant circulant dans l'électrode.

6. Procédé selon l'une quelconque des revendications précédentes, dans lequel l'électrode est immergée dans une suspension de mitochondries ou de chloroplastes.

7. Procédé selon l'une quelconque des revendications précédentes, dans lequel l'électrode est immergée dans une suspension de cellules intactes.

8. Procédé selon l'une quelconque des revendications précédentes, dans lequel l'électrode est placée dans un milieu qui est au contact d'un tissu à cellules connectées.